# EUROPÄISCHE PATENTANMELDUNG

(11) **EP 3 591 406 A1**
(43) Veröffentlichungstag der Anmeldung: **08.01.2020**
(21) Anmeldenummer: 18182267.7
(22) Anmeldetag: 06.07.2018
(51) Int. Cl.: G01N 33/58, G01N 33/68, G01N 33/543, G01N 33/564

(54) **VORRICHTUNG UND VERFAHREN ZUR ANTIKÖRPERDETEKTION**

(71) Anmelder: Euroimmun Medizinische Labordiagnostika AG, 23560 Lübeck (DE)
(72) Erfinder: Morgenroth, Katja, 23923 Herrnburg (DE); Viertel, Vanessa, 23554 Lübeck (DE); Steller, Ulf, 21244 Buchholz (DE); Gerlach, Stefan, 23627 Groß Grönau (DE); Marzahl, Christian, 23552 Lübeck (DE); Voigt, Jörn, 23558 Lübeck (DE); Stöcker, Winfried, 23627 Groß Grönau (DE)

(57) **Zusammenfassung**

Beschrieben ist ein Antigen-Chip (5), aufweisend eine ebene Substratoberfläche (9); und Antigen-Spots (11a, ..., 11e), die auf der Substratoberfläche in einem vorbestimmten Muster aufgebracht sind und einen ersten Fluoreszenzfarbstoff enthalten. Ferner sind einen Vorrichtung und ein Verfahren zum Detektieren von Antikörpern in einer Probe bereitgestellt. Noch ferner betrifft die Anmeldung ein Kit zur Verwendung in einem Verfahren zum Detektieren von Antikörpern in einer Probe.

## Beschreibung

Die vorliegende Anmeldung betrifft einen Antigen-Chip bzw. Antigen-Substrat zur Antikörperdetektion und eine Vorrichtung zum Detektieren von Antikörpern in einer Probe, welche den Antigen-Chip aufweist. Ferner betrifft die vorliegende Anmeldung ein Verfahren zur Herstellung eines Antigen-Chips/Substrats sowie ein Verfahren zum Detektieren von Antikörpern in einer Probe. Noch ferner betrifft die Anmeldung ein Kit zur Verwendung in einem Verfahren zum Detektieren von Antikörpern in einer Probe.

Herkömmlicherweise wird indirekte Immunfluoreszenztechnologie verwendet, um Antikörper in flüssigen Proben zu identifizieren. Dazu können zum Beispiel Zellen, Gewebeschnitte oder aufgereinigte, biochemisch charakterisierte Substanzen als Antigensubstrate verwendet werden. In einem ersten Inkubationsschritt binden in den Proben enthaltene nachzuweisende (primäre) Antikörper an die Festphasen-gebundenen Antigene. In einem zweiten Inkubationsschritt werden an diese aus der Probe gebundenen Antikörper (sekundäre) Fluoreszenz-markierte Anti-Human-Antikörper gebunden. In einem Fluoreszenzmikroskop können dann die gebundenen sekundären Antikörper aufgrund ihrer Fluoreszenzmarkierung detektiert werden, welche die Anwesenheit von für jedes Antigen spezifischen primären Antikörpern in der Probe anzeigen.

WO 2012/094427 A1 offenbart Verfahren für Fluoreszenzdetektion von Antikörpern durch Fluoreszenzdetektion. US 2005/0124017 offenbart Fluoreszenzbildgebung zur Detektion von Proteinen, Antikörpern, Drogen oder anderen Liganden in einer Probe, wobei ein Bild abgetastet wird. US 2004/0253640 offenbart ein Microarray mit darauf gedruckten Proteinen, um ein Zielprotein durch Immunofluoreszenz zu detektieren. WO 2017/025954 A1 offenbart einen Antigen-Chip für Immunofluoreszenzdetektion, wobei der Antigen-Chip zur Detektion abgetastet wird. WO 2012/052994 A2 offenbart Microarrays zur Hochdurchsatzcharakterisierung der Immunantwort. WO 2012/037369 A offenbart Antikörperdetektion durch Fluoreszenzdetektion. WO 2004/027379 A offenbart eine Technik von rollenden Kugeln, um primäre Antikörper zu detektieren, welche auf einem Antigen-Microarray gebunden sind. WO 2000/063701 A2 offenbart Microarrays von Polypeptiden, um zum Beispiel Antikörper zu detektieren, wobei Fluoreszenzdetektion verwendet wird und die Microarrays abgetastet werden.

US 2017/0016052 A1 offenbart Array-Systeme mit internen Kontrollen.

WO 2011/101487 A1 offenbart ein Verfahren zur Krankheitsdiagnose über eine simultane Detektion von Antikörpern, welche an synthetische und zelluläre Substrate gebunden sind, wobei die Antikörper durch indirekte Immunofluoreszenz detektiert werden. Ein synthetisches Substrat ist ein Mikropartikel oder eine Kugel, welche mit einem aufgereinigten nativen Antigen oder einem rekombinanten Antigen beschichtet ist, ein Fluoreszenzmikroskop ist mit einer Kamera und mit einem Abtastsystem ausgestattet.

EP 2 362 222 offenbart ein Verfahren zur Krankheitsdiagnose, wobei simultan Antikörper detektiert werden, welche an zelluläre oder Gewebesubstrate gebunden sind, oder welche an synthetische Substrate gebunden sind, wie etwa Mikropartikel oder Kugeln, welche mit den spezifischen Antigenen beschichtet sind. Dabei wird Vielfarbfluoreszenzmikroskopie benutzt, um die gebundenen Antikörper zu detektieren, wobei die Substrate und die gebundenen Antikörper verschiedene Fluoreszenzfarben aufweisen.

Es ist beobachtet worden, dass die herkömmlichen Systeme und Verfahren zur Detektion von Antikörpern nicht unter allen Umständen und in einer akzeptablen Messzeit eine zuverlässige Detektion einer Vielzahl von Antikörpern ermöglichen. Auch erfordern herkömmliche Verfahren eine relativ große Menge an Antigen oder Probe. Ferner sind herkömmliche Verfahren für Hochdurchsatzanwendungen in der medizinischen Routinediagnostik nur bedingt geeignet.

Eine Aufgabe der vorliegenden Erfindung ist es somit, Systeme und Verfahren bereitzustellen, welche eine zuverlässige Detektion einer Vielzahl von Antikörpern in einer flüssigen Probe unterstützen, insbesondere für die medizinische Routinediagnostik geeignet sind und ferner insbesondere zumindest semi-quantitative Ergebnisse liefern.

Die Aufgabe wird durch die Gegenstände der unabhängigen Ansprüche gelöst, welche auf einen Antigen-Chip, auf eine Vorrichtung zum Detektieren von Antikörpern in einer Probe, auf ein Verfahren zur Herstellung eines Antigen-Chips und auf ein Verfahren zum Detektieren von Antikörpern in einer Probe gerichtet sind. Die Aufgabe wird ferner durch ein Kit zur Verwendung in einem Verfahren zum Detektieren von Antikörpern in einer Probe gelöst. Die abhängigen Ansprüche spezifizieren besondere Ausführungsformen der vorliegenden Erfindung.

Gemäß einer Ausführungsform der vorliegenden Erfindung ist bereitgestellt ein Antigen-Chip, der eine ebene Substratoberfläche und Antigen-Spots aufweist, die auf der Substratoberfläche in einem vorbestimmten Muster aufgebracht sind und einen Luminophor, ein chromogenes Substrat oder ersten Farbstoff, insbesondere ersten Fluoreszenzfarbstoff enthalten.

Der Chip kann als ein festes Substrat aufgefasst werden, etwa eine Glasplatte oder zum Beispiel Siliziumplatte. Der Chip kann auch aus Kunststoff oder auch aus Metall gefertigt sein. Der Chip kann lichtdurchlässig oder nicht lichtdurchlässig sein, um Durchlicht- oder Auflichtbeleuchtung bzw. - detektion zu unterstützen. Der Antigen-Chip kann in einem Scanner- oder Kamerasystem, insbesondere Fluoreszenzmikroskop, zur Untersuchung einer Probe eingesetzt werden und kann dann mit Beleuchtungslicht bzw. Anregungslicht bestrahlt werden. Durch das Beleuchtungslicht bzw. Anregungslicht kann in den Antigen-Spots der erste Farbstoff, insbesondere Fluoreszenzfarbstoff, zum Aussenden von Licht, das heißt zur ersten Strahlung (z.B. von dem ersten Farbstoff reflektiertem Beleuchtungslicht), insbesondere ersten Fluoreszenz, angeregt werden. Ist an einen bestimmten Antigen-Spot ferner ein Antikörper aus der Probe gebunden und wurden die Antigen-Spots bzw. der gesamte Antigen-Chip ferner mit sekundären fluoreszenzmarkierten Antikörpern inkubiert, so wird bevorzugt auch der zweite Fluoreszenzfarbstoff (welcher an den sekundären Antikörper gebunden ist) zur Aussendung von Licht angeregt, das heißt zur zweiten Fluoreszenz.

Die erste Fluoreszenz kann Aussendung von Licht in einem ersten Wellenlängenbereich umfassen und die zweite Fluoreszenz kann Aussendung von Licht in einem zweiten Wellenlängenbereich umfassen, wobei der erste Wellenlängenbereich und der zweite Wellenlängenbereich nur einen geringen oder im Wesentlichen keinen Überlappungsbereich aufweisen, etwa einen Überlappungsbereich oder eine Überlappung, welcher zum Beispiel weniger als 10%, insbesondere weniger als 5%, eines Integrals einer Emission der ersten Fluoreszenz über die Wellenlänge entspricht. Eine ähnliche Definition kann in Bezug auf die zweite Fluoreszenz gegeben sein. Die Anregung der ersten und der zweiten Fluoreszenz kann über die gleiche oder verschiedene Wellenlängen bzw. Wellenlängenbereiche erfolgen.

Die Antigen-Spots können auf die ebene Substratoberfläche gedruckt bzw. gespottet sein. Dazu kann eine Piezodrucktechnik verwendet werden, wie sie im Stand der Technik bereits verwendet wird. Das vorbestimmte Muster kann zum Beispiel die Anordnung, die Größe und/oder die Form der Antigen-Spots definieren. Das vorbestimmte Muster kann während des Durchführens eines Verfahrens zum Detektieren von Antikörpern in einer Probe zur Lokalisierung der Spots und/oder zur Identifizierung der jeweiligen in den Antigen-Spots enthaltenen Antigene verwendet werden. Zum Beispiel können Antigen-Spots, welche das gleiche Antigen enthalten, mehrfach auf dem Antigen-Chip vorkommen und entlang einer Linie mit einem spezifischen Spotabstand angeordnet sein und/oder eine bestimmte Form und/oder eine bestimmte Größe aufweisen. Antigen-Spots, welche ein anderes Antigen enthalten, können ebenfalls auf einer Linie angeordnet sein, können jedoch einen anderen (oder einen gleichen) Spotabstand aufweisen, können eine andere (oder eine gleiche) Form aufweisen und können eine andere (oder eine gleiche) Größe aufweisen.

Der Antigen-Chip kann in einem Verfahren zum Detektieren von Antikörpern in einer Probe gemäß einer Ausführungsform der vorliegenden Erfindung eingesetzt werden.

Der Luminophor, das chromogene Substrat oder erste Farbstoff, insbesondere Fluoreszenzfarbstoff, kann ein Lokalisieren der Antigen-Spots durch Detektion der ersten Strahlung, insbesondere Fluoreszenzdetektion der ersten Fluoreszenz, nach Bestrahlen mit Beleuchtungslicht, insbesondere Anregung durch Anregungslicht, erlauben bzw. erleichtern. Selektiv an den bestimmten Positionen der Antigen-Spots kann dann die zweite Chemilumineszenz oder Fluoreszenz, welche durch Aussendung von Licht nach Anregung des zweiten Fluoreszenzfarbstoffes erfolgt, detektiert werden. Etwaig außerhalb aller Antigen-Spots an die ebene (bloße) Substratoberfläche gebundene primäre und/oder sekundäre Antikörper können dann als Messuntergrund erkannt werden. Von dem Messuntergrund herrührende Fluoreszenzsignale können dann zum Beispiel von den Fluoreszenzsignalen abgezogen werden, welche von Antigen-Spots herrühren, um somit die Messgenauigkeit zu erhöhen.

Gemäß einer Ausführungsform der vorliegenden Erfindung ist der Antigen-Chip derart ausgebildet, dass Antigen-Spots, die verschiedene Antigene enthalten, verschiedene Mengen bzw. Konzentrationen des Luminophors, chromogenen Substrats oder ersten Farbstoffes, insbesondere Fluoreszenzfarbstoffes, derart enthalten, dass davon zurückgeworfene erste Strahlung, insbesondere angeregte erste Fluoreszenz, unterschiedliche Intensität oder im Wesentlichen gleiche Intensität hat. Beispielsweise kann eine (oder mehrere) Reihe(n) von Antigen-Spots einen bestimmten Antigen-SpotAbstand aufweisen, so dass die Reihe(n) gegenüber anderen Reihen von Antigen-Spots unterschieden werden können und so eine oder mehrere Referenzreihen entstehen. Ein Unterscheidungsmerkmal der Antigen- / Kontrolllinien untereinander kann eine unterschiedliche Intensität der ersten Fluoreszenz sein. Z.B. kann von Antigen-Spots verschiedener Linien zurückgeworfene erste Strahlung, insbesondere angeregte erste Fluoreszenz, im Wesentlichen unterschiedliche Intensität haben, um die Linien unterscheiden und/oder identifizieren zu können.

Wenn die Antigen-Spots, welche verschiedene Antigene enthalten, im Wesentlichen die gleiche (oder vorbestimmte unterschiedliche) Intensität der ersten Fluoreszenz nach Anregung durch Anregungslicht aussenden, kann eine Lokalisierung der Antigen-Spots nach einheitlichem Verfahren zuverlässiger durchgeführt werden. Auch die Belichtung zur Aufnahme eines ersten Bildes kann dann einfacher eingestellt werden und es kann insbesondere auf mehrere Aufnahmen des ersten Bildes mit verschiedenen Belichtungszeiten verzichtet werden. Damit kann das Verfahren beschleunigt und zuverlässiger durchgeführt werden.

Die Antigene, welche in den verschiedenen Antigen-Spots enthalten sind, können durch Bindung von Antikörpern Autoimmunerkrankungen, Allergien und Infektionskrankheiten nachweisen. Im vorliegenden Testsystem kann es sich insbesondere um antinukleäre und extrahierbare antinukleäre Antikörper aus dem Bereich der Kollagenosen handeln.

Die Antigene, welche in den verschiedenen Antigen-Spots enthalten sind, können ausgewählt und ausgebildet sein, Antikörper, die sich als Folge von Autoimmunerkrankungen, Allergien und Infektionskrankheiten bilden zu binden. Im vorliegenden Testsystem betrifft dies insbesondere antinukleäre und/oder extrahierbare antinukleäre Antikörper aus dem Bereich der Kollagenosen. Insbesondere können beispielsweise folgende Antigene (allein oder in beliebiger Kombination) in den Antigen-Spots enthalten sein: RNP/Sm, Sm, Scl-70, Rib.P0, Jo-1, SS-A, SS-B, dsDNA, Nukleosomen/Chromatin, Cenp-B, RNP A,C,68kDa, Ro-52, Ku, Histone, DFS70. Die diagnostizierbaren Kollagenosen können zumindest eine der folgende Formen enthalten: SLE (Systemic lupus erythematosus), PM, DM (Myositis), SS (Sjögren's Syndrom), CREST-Syndrom (limited cutaneous form of systemic sclerosis IcSSc), PSS (progressive systemic sclerosis), MCTD (mixed connective tissue disease, sharp syndrome), AID (autoimmune induced disease). Damit können verschiedene Krankheiten diagnostiziert werden.

Der Ausdruck "Chemilumineszenz", wie hierin verwendet, bezieht sich auf eine chemische Reaktion, bei der Energie spezifisch zu einem Molekül geleitet wird, was bewirkt, dass es elektronisch angeregt wird und anschließend ein Photon freisetzt, wodurch sichtbares Licht emittiert wird. Wärmeenergie wird für diese Reaktion nicht benötigt. Chemilumineszenz beinhaltet somit die direkte Umwandlung von chemischer Energie in Lichtenergie. Bevorzugt entsteht Chemilumineszenz durch die Reaktion eines Luminophors mit anderen Verbindungen. Diese Reaktionen können durch Enzyme katalysiert sein. In weiter bevorzugten Ausführungsformen der Erfindung ist der Luminophor aus der Gruppe bestehend aus Luminol und Derivate davon, Acridin und Derivate davon und Luciferine ausgewählt. Diese Verbindungen können durch verschiedene enzymatische Reaktionen zur Chemilumineszenz angeregt werden. Die entsprechenden Verbindungen und Reaktionen sind im Stand der Technik bekannt. In weiter bevorzugten Ausführungsformen der Erfindung ist das Luminophor Luminol oder ein Derivat davon, wobei durch eine von Peroxidase, insbesondere Meerettichperoxidase, katalysierte Reaktion Licht emittiert wird (Chemilumineszenz). In alternativen Ausführungsformen der Erfindung ist das Luminophor Acridin oder ein Derivat davon, insbesondere ein Acridiniumester oder Acridiniumsulfonamid, wobei durch eine von Phosphatase, insbesondere alkalische Phosphatase (AP), katalysierte Reaktion Licht emittiert wird (Chemilumineszenz). In weiteren alternativen Ausführungsformen der Erfindung ist das Luminophor ein Luciferin, insbesondere D-Luciferin, wobei durch eine von Luciferase katalysierte Reaktion Licht emittiert wird (Chemilumineszenz).

Ein "chromogenes Substrat", im Sinne der vorliegenden Erfindung, ist ein Reagenz, das zur Messung von Enzymaktivitäten eingesetzt werden kann. Das chromogene Substrat wird durch enzymatische Aktivität so verändert, dass ein direktes oder indirektes Produkt der enzymatischen Reaktion (photometrisch) quantifiziert werden kann. Das chromogene Substrat besteht z.B. aus einem Oligopeptid, an das ein Azofarbstoff, zum Beispiel Paranitroanilin, gekoppelt ist. Das Peptid imitiert die Schnittstelle, an der das zu untersuchende Enzym sein physiologisches Substrat spaltet. Durch die Enzymwirkung wird der Farbstoff freigesetzt. Die Farbentwicklung kann photometrisch quantifiziert und über eine Eichkurve die Enzymaktivität ermittelt werden. Das chromogene Substrat kann allerdings auch 5-Brom-4-chlor-3-indoxylphosphat (BCIP) und/oder Nitroblautetrazoliumchlorid (NBT) sein. Ein chromogenes Substrat kann z.B. kolorimetrisch nachgewiesen werden.

Gemäß einer Ausführungsform der vorliegenden Erfindung ist der Antigen-Chip derart ausgebildet, dass Antigen-Spots eines gleichen Antigens jeweils in einer Linie, insbesondere mit einem vorbestimmten Spotabstand, angeordnet sind, wobei verschiedenen Antigenen zugeordnete Linien insbesondere einen vorbestimmten Linienabstand aufweisen, wobei ferner insbesondere der Linienabstand zweier Linien größer ist als der Spotabstand innerhalb einer Linie. Alternativ kann der Abstand zwischen zwei Linien aber auch kleiner sein als der Spotabstand innerhalb einer Linie.

Ein Auswertesystem einer Vorrichtung zum Detektieren von Antikörpern in einer Probe kann zum Beispiel ausgebildet sein, einen Linienabstand zwischen zwei Linien von Antigen-Spots zu bestimmen und/oder einen Spotabstand von Antigen-Spots innerhalb einer Linie zu bestimmen. Der Spotabstand kann spezifisch für Antigen-Spots sein, welche ein bestimmtes Antigen enthalten. Daher kann aus dem bestimmten Spotabstand auf dasjenige Antigen geschlossen werden, welches in den Antigen-Spots der untersuchten Linie enthalten ist. Der Linienabstand einer ersten Linie von Antigen-Spots zu einer zweiten Linie von Antigen-Spots kann ebenfalls bestimmt werden, um diejenigen Antigene bzw. dasjenige Antigen zu bestimmen, welches in den Antigen-Spots der zweiten Linie enthalten ist, welche der ersten Linie benachbart ist, für die das Antigen bereits bestimmt wurde, welches in den Antigen-Spots enthalten ist. Ein bestimmter Linienabstand und ein bestimmter Spotabstand kann kombiniert werden, um eine zuverlässige Lokalisierung der Antigen-Spots und/oder eine Zuordnung der Antigen-Spots zu bestimmten Antigenen zu bestimmen.

Die Antigen-Spots können verschiedene Formen haben, insbesondere können sie im Wesentlichen kreisförmig sein und können im Wesentlichen eine unterschiedliche Größe, insbesondere einen im Wesentlichen gleichen oder unterschiedlichen Durchmesser, aufweisen. Damit können herkömmliche Spotting-Techniken verwendet werden, um die Antigen-Spots auf die Substratoberfläche aufzubringen.

Gemäß einer Ausführungsform der vorliegenden Erfindung ist der Antigen-Chip derart ausgebildet, dass auf der Substratoberfläche ferner zumindest ein, zwei, insbesondere mindestens drei, Spots oder Linien von Antigen-Spots (auch Kalibrierspots oder interner Kalibrator genannt) aufgebracht sind, die einen primären Antikörper (z.B. IgG) in identischen oder verschiedenen Mengen bzw. Konzentrationen enthalten. Nach Inkubation mit einem fluoreszenzmarkierten sekundären Antikörper (welcher mit dem zweiten Fluoreszenzfarbstoff markiert ist) kann auch von den Kalibrierspots, die den primären Antikörper enthalten, eine zweite Fluoreszenz ausgehen und zwar, entsprechend den verschiedenen Mengen bzw. Konzentrationen des primären Antikörpers, in verschiedenen Intensitäten. Dadurch kann durch Auswertung der Intensitäten der zweiten Fluoreszenz eine Kalibrierung von einem Auswertesystem durchgeführt werden, um eine Quantifizierung von an andere Antigen-Spots gebundenen Antikörpern zu erlauben. Beispielsweise können die verschiedenen Mengen bzw. Konzentrationen vorbestimmt bzw. bekannt sein. Die von den verschiedenen Kalibrierungsspots detektierten Intensitäten der zweiten Fluoreszenz können den jeweiligen Mengen bzw. Konzentrationen zugeordnet werden und es kann eine Extrapolation bzw. Interpolation auf Zwischenkonzentrationen bzw. auf Konzentrationen vorgenommen werden, welche größer oder kleiner sind als die Konzentrationen, welche in den Kalibrierungsspots enthalten sind. Damit kann der Antigen-Chip eine quantitative bzw. zumindest eine semi-quantitative Analyse von Antikörpern in einer Probe unterstützen. Für eine präzise Quantifizierung kann ein externer Kalibrator (in Form eines Kalibratorserums) eingesetzt werden.

Gemäß einer Ausführungsform der vorliegenden Erfindung ist der Antigen-Chip derart ausgebildet, dass auf dem Substrat ferner zumindest eine Linie, insbesondere eine Linie eines Antigens mit geringerem Dotabstand im Vergleich zu den Dotabständen der anderen Linien des Antigen-Chips, als Positivkontrolle aufgebracht ist. In einer Ausführungsform kann diese Linie als eine durchgehende Linie ausgeprägt sein. Mit anderen Worten ist gemäß einer Ausführungsform der vorliegenden Erfindung der Antigen-Chip derart ausgebildet, dass auf dem Substrat Spots in unterschiedlichen Abständen bis hin zu einer durchgehenden Linie ausgeprägt sind.

Gemäß einer weiteren Ausführungsform können Antigen-Spots, die z.B. das gleiche Antigen enthalten, entlang einer Linie angeordnet sein, wobei ihr Spotabstand so gering ist, dass die verschiedenen Spots miteinander in Kontakt stehen und als durchgehende Linie wahrgenommen werden. Der Antigen-Chip der vorliegenden Erfindung umfasst also Chips, die ausschließlich aus Spots, ausschließlich aus Linien oder aus einer Kombination von Spots und Linien bestehen.

Die Positivkontrolle (z.B. anti-human IgG) kann dazu verwendet werden, festzustellen, ob der betreffende Antigen-Chip überhaupt mit einer Probe inkubiert worden ist. Jede erwartete Probe kann zum Beispiel einen oder mehrere bestimmte Antikörper in jedem Fall enthalten, auch wenn keine pathologische Bedingung vorliegt. Auch die Position bzw. die Lokalisierung der Positivkontrolle kann durch das Auswertesystem bestimmt werden und kann insbesondere zur Bestimmung der Orientierung des Antigen-Chips herangezogen werden. Zur Orientierung der Antigene auf dem Chip kann beispielsweise der einzigartige Spotabstand innerhalb der Linien und/oder die Anordnung der Linien mit unterschiedlichem Spotabstand zueinander dienen. Damit kann eine Analyse von Antikörpern zuverlässiger durchgeführt werden.

Der erste Fluoreszenzfarbstoff kann zum Beispiel in einem roten Wellenlängenbereich fluoreszieren, zum Beispiel in einem Bereich zwischen 550 nm und etwa 800 nm mit einem Maximum zwischen 650 nm und 700 nm. Dazu kann beispielsweise der erste Fluoreszenzfarbstoff DY521XL Verwendung finden. Die Anregung des ersten Fluoreszenzfarbstoffes kann in einem Frequenzbereich zum Beispiel zwischen 450 nm und 650 nm mit einem Maximum zum Beispiel zwischen 500 nm und 540 nm erfolgen. Die Anregung des zweiten Fluoreszenzfarbstoffes kann im Wesentlichen in demselben Wellenlängenbereich wie die Anregung des ersten Fluoreszenzfarbstoffes erfolgen. Damit ist in einer Vorrichtung zur Detektion von Antikörpern in einer Probe beispielsweise nur eine einzige Beleuchtungslichtquelle zur Erzeugung des Anregungslichts erforderlich, welche relativ schmalbandig ausgeführt sein kann. Damit kann vorteilhaft das Anregungslicht herausgefiltert werden, bevor die erste Fluoreszenz durch eine erste Kamera und die zweite Fluoreszenz durch eine zweite Kamera oder beide Fluoreszenzsignale durch die erste oder die zweite Kamera (z.B. nach einem Filterwechsel) detektiert werden, ohne im Wesentlichen störendes Anregungslicht zu detektieren.

Gemäß einer Ausführungsform der vorliegenden Erfindung ist die Substratoberfläche zur Fokussierung mikrostrukturiert. Die Mikrostrukturierung kann durch eine Oberflächenbehandlung (zum Beispiel Aufrauung) ausgeführt sein und kann eine Fokussierung auf die Substratoberfläche erleichtern. Verschiedene Verfahren zur Mikrostrukturierung, wie beispielsweise Spritzgießen, Heißprägen oder Imprint-Verfahren, sind im Stand der Technik bekannt. Es können verschiedene Mikrostruktur-Marker auf der Oberfläche aufgebracht sein, welche auch zur Identifizierung des Antigen-Chips, zur Identifizierung der Probe etc. verwendet werden können.

Das Antigen-Substrat (der Chip) umfasst insbesondere eine Fläche mit einer Größe zwischen 1 mm x 1 mm und 2 mm x 4 mm.

Gemäß einer Ausführungsform umfasst keiner der Antigen-Spots ein Gel oder einen Gel-Spot oder Gel-Pad, insbesondere ein (3D-) Polymer-Gel und/oder ein Polyacrylamid-Gel, in denen die Antigene gebunden sind. Gemäß einer Ausführungsform umfassen die Antigen-Spots lediglich, den ersten Farbstoff, das chromogene Substrat oder Luminophor, das Antigen und (getrockneten) Puffer ohne Polymer und/oder haben eine Dicke zwischen 0,1 µm und 5 µm.

Es wird darauf hingewiesen, dass Merkmale, die im Zusammenhang mit einem Antigen-Chip individuell oder in irgendeiner Kombination beschrieben, erläutert oder bereitgestellt sind, ebenso, individuell oder in irgendeiner Kombination, auf eine Vorrichtung zum Detektieren von Antikörpern in einer Probe angewendet werden können, auf ein Verfahren zur Herstellung eines Antigen-Chips angewendet werden können und auch auf ein Verfahren zum Detektieren von Antikörpern in einer Probe angewendet werden können und umgekehrt, gemäß Ausführungsformen der vorliegenden Erfindung.

Gemäß einer Ausführungsform der vorliegenden Erfindung ist bereitgestellt eine Vorrichtung zum Detektieren von Antikörpern in einer Probe, aufweisend: einen Antigen-Chip gemäß einer der vorangehenden Ausführungsformen; und ein Scanner- oder Kamerasystem mit einer Beleuchtungsquelle (z.B. ein Fluoreszenzmikroskop), die ausgebildet ist, Beleuchtungslicht, insbesondere Anregungslicht einer ersten Wellenlänge, zu erzeugen, und die angeordnet ist, den Antigen-Chip zu beleuchten, insbesondere zu bestrahlen; einer ersten Kamera, die zum Aufnehmen eines ersten Bildes durch Detektion von von der Probe ausgehender durch das Beleuchtungslicht hervorgerufener erster Strahlung, insbesondere durch das Anregungslicht angeregter erster Fluoreszenz, ausgebildet ist und/oder zum Aufnehmen eines zweiten Bildes durch Detektion von von der Probe ausgehender Chemilumineszenz oder durch das Beleuchtungslicht, insbesondere Anregungslicht, angeregter zweiter Fluoreszenz ausgebildet ist.

Das Scanner- und/oder Kamerasystem, insbesondere das Fluoreszenzmikroskop (55), kann ferner eine zweite Kamera aufweisen, die zum Aufnehmen eines zweiten Bildes durch Detektion von von der Probe ausgehender Chemilumineszenz oder durch das Anregungslicht angeregter zweiter Fluoreszenz ausgebildet ist, insbesondere wenn die erste Kamera die Detektion der zweiten Fluoreszenz nicht leisten kann.

Das Scanner- oder Kamerasystem zur Erfassung von Chemilumineszenz oder Fluoreszenzen, z.B. das Fluoreszenzmikroskop, kann eine Objekthalterung umfassen, auf welcher der Antigen-Chip gehaltert werden kann. Das Fluoreszenzmikroskop kann weiter ein Objektiv umfassen, welches eine oder mehrere Linsen aufweisen kann, mit denen ein vergrößertes Bild des Antigen-Chips oder zumindest eines Teils des Antigen-Chips erzeugt werden kann, wenn die Beleuchtungsquelle den Antigen-Chip beleuchtet. Das Fluoreszenzmikroskop kann ferner zumindest einen Strahlteiler (zum Beispiel halbdurchlässigen Spiegel) umfassen, um einen Teil des von dem Antigen-Chips ausgehenden (Fluoreszenz)Lichts auf die erste Kamera zu richten und einen anderen Teil auf die zweite Kamera zu richten. Die Vorrichtung kann ausgebildet werden, das erste Bild gleichzeitig mit dem oder zeitversetzt zum zweiten Bild aufzunehmen. Während der Aufnahme des ersten Bildes und der Aufnahme des zweiten Bildes kann die Beleuchtungsquelle das Anregungslicht auf den Antigen-Chip richten. Das Anregungslicht regt den ersten Fluoreszenzfarbstoff zur Aussendung der ersten Fluoreszenz an. In bevorzugten Ausführungsformen regt es auch einen zweiten Fluoreszenzfarbstoff (welcher insbesondere an einen sekundären Antikörper gebunden ist) zur Aussendung von zweiter Fluoreszenz an. Somit braucht die Vorrichtung nur eine einzelne Beleuchtungsquelle aufzuweisen, um ein Verfahren zum Detektieren von Antikörpern in einer Probe unter Verwendung des Antigen-Chips, welcher oben beschrieben wurde, durchzuführen. Die Beleuchtungsquelle kann zum Beispiel einen Laser, eine LED, insbesondere cLED, umfassen. Andere Lichtquellen sind möglich.

Die Vorrichtung kann ferner ein erstes Filter, aufweisen welches in einem ersten Strahlengang stromaufwärts der ersten Kamera angeordnet ist und ausgebildet ist, Anregungslicht und/oder Licht der zweiten Fluoreszenz im Wesentlichen zu eliminieren, und insbesondere ferner ein zweites Filter aufweisen, welches in einem zweiten Strahlengang stromaufwärts der zweiten Kamera angeordnet ist und ausgebildet ist, Anregungslicht und Licht der ersten Fluoreszenz im Wesentlichen zu eliminieren. Das erste Filter kann einen ersten Wellenlängenbereich um ein Maximum der ersten Fluoreszenz passieren lassen, kann jedoch einen zweiten Wellenlängenbereich um das Maximum der zweiten Fluoreszenz im Wesentlichen eliminieren. Damit kann eine Lokalisierung der Antigen-Spots zuverlässig durchgeführt werden. Umgekehrt kann das zweite Filter im Wesentlichen einen zweiten Wellenlängenbereich um das Maximum der Fluoreszenz der zweiten Fluoreszenz passieren lassen, während Licht des ersten Wellenlängenbereichs um ein Maximum der ersten Fluoreszenz im Wesentlichen eliminiert bzw. abgeschwächt wird. Damit kann zuverlässig die Menge von Antikörpern bestimmt werden, welche an die verschiedenen Antigen-Spots bzw. an die darin enthaltenen Antigene binden. Der erste Strahlengang bzw. die erste Fluoreszenz kann zum Beispiel rotes Licht enthalten und wird bisweilen auch als Rotkanal bezeichnet. Der zweite Strahlengang bzw. die zweite Fluoreszenz kann zum Beispiel grünes Licht enthalten und wird bisweilen auch als Grünkanal bezeichnet. In anderen Ausführungsformen ist in dem ersten Strahlengang bzw. dem zweiten Strahlengang bzw. in der ersten Fluoreszenz und in der zweiten Fluoreszenz Licht anderer Wellenlängen umfasst, solange jedoch die entsprechenden Wellenlängenbereiche hinreichend voneinander getrennt sind bzw. wenig überlappen. Das System kann eine (einzige) Anregungswellenlänge (bzw. Anregungswellenlängenbereich) für beide Fluoreszenzfarbstoffe nutzen. Gemäß einer anderen Ausführungsform werden die beiden Fluoreszenzfarbstoffe mit unterschiedlichen Wellenlängen bzw. Wellenlängenbereichen (z.B. mit unterschiedlichen Lichtquellen) angeregt.

In einer bevorzugten Ausführungsform der Vorrichtung ist diese so ausgebildet, dass Fluoreszenz angeregt und detektiert werden kann, wobei die erste und zweite Fluoreszenz über die gleiche Wellenlänge anregbar sind. In weiter bevorzugten Ausführungsformen wird die erste Fluoreszenz durch Anregung von DY-521-XL und die zweite Fluoreszenz durch Anregung von Fluoresceinisothiocyanat (FITC) erzeugt.

Die Vorrichtung kann gemäß einer Ausführungsform der vorliegenden Erfindung ferner ein Auswertesystem aufweisen, welches ausgebildet ist, basierend aus dem ersten Bild Positionen der Antigen-Spots zu bestimmen, die Positionen der Antigen-Spots jeweiligen Antigenen anhand des vorbestimmten Musters zuzuordnen, und an Antigen-Spots gebundene Antikörper basierend auf dem zweiten Bild, insbesondere zumindest semi-quantitativ, zu erkennen.

Das erste Bild kann auch als ein Rotbild bezeichnet werden und das zweite Bild kann auch als ein Grünbild bezeichnet werden. Die erste Kamera und auch die (optionale) zweite Kamera können dabei jeweils ein lichtempfindliches Feld (zum Beispiel zweidimensionales Feld) von lichtempfindlichen Elementen, etwa Fotodioden, umfassen oder kann zum Beispiel als ein CMOS-Sensorfeld ausgebildet sein. Die erste Kamera und auch die zweite Kamera können dabei zum Beispiel eine geeignete Abbildungsoptik aufweisen.

Das Auswertesystem kann ausgebildet sein, Bildverarbeitung durchzuführen. Das Auswertesystem kann zum Beispiel durch Software gesteuert sein und kann einen Prozessor aufweisen, welcher ausgebildet ist, die Software auszuführen. Die von dem Auswertesystem bestimmten Positionen der Antigen-Spots können mit Positionen des vorbestimmten Musters korreliert werden, um zum Beispiel Orientierung und Vergrößerung am Mikroskop zu bestimmen und aufeinander abzustimmen. Zum Beispiel kann das Auswertesystem ausgebildet sein, Intensitäten in dem zweiten Bild über Bereiche zu integrieren (bzw. zu mitteln), über welche in dem ersten Bild ein Antigen-Spot bestimmt worden ist. Von diesen integrierten oder gemittelten Intensitäten können dann zum Beispiel Hintergrundfluoreszenzen/Untergrundintensitäten, das heißt Intensitäten über Bereiche des zweiten Bildes, welche außerhalb von in dem ersten Bild bestimmten Antigen-Spots liegen, abgezogen werden. Das Auswertesystem kann somit ausgebildet sein, das zweite Bild auszuwerten, um Intensitäten der von den einzelnen Antigen-Spots ausgesandten zweiten Fluoreszenz zu bestimmen, und insbesondere Intensitäten mehrerer Antigen-Spots, die das gleiche Antigen enthalten, zu mitteln. Die Mittelung von Intensitäten, welche von Antigen-Spots herrühren, welche das gleiche Antigen enthalten, kann die Messgenauigkeit erhöhen.

Gemäß einer Ausführungsform der vorliegenden Erfindung ist das Auswertesystem ferner ausgebildet, in dem zweiten Bild Intensitäten von von zumindest einem, zwei oder drei oder mehr Kalibrierungsspots bzw. Kalibrierungsspotreihen ausgehender zweiter Fluoreszenz zu bestimmen, um eine Kalibrierung durchzuführen, wobei die Kalibrierungsspots bzw. Kalibrierungsspotreihen einen primären Antikörper (z.B. IgG) in verschiedenen Mengen bzw. Konzentrationen enthalten. Die Mengen bzw. Konzentrationen des primären Antikörpers in den Kalibrierungsspots können vorbekannt oder vordefiniert sein. Von dem Auswertesystem kann durch Detektion der von den Kalibrierungsspots bzw. Kalibrierungsspotreihen ausgehenden Intensitäten eine Abhängigkeit von Konzentration bzw. Menge und erhaltener Intensität abgeleitet werden. Beispielsweise kann die Menge an Antikörpern aus verschiedenen Proben über den Krankheitsverlauf von einem gegebenen Patienten relativ zu einander bestimmt werden. Umgekehrt kann dann aus einer detektierten Intensität der zweiten Fluoreszenz, welche von einem bestimmten Antigen-Spot ausging, auf die Konzentration bzw. Menge des an diesen Antigen-Spot gebundenen Antikörpers geschlossen werden, um eine quantitative oder zumindest eine semi-quantitative Auswertung zu ermöglichen.

Gemäß einer Ausführungsform der vorliegenden Erfindung ist das Auswertesystem ferner ausgebildet, in dem ersten Bild zumindest eine Linie, insbesondere eine Linie mit geringerem Dotabstand im Vergleich zu den Dotabständen der anderen Linien des Antigen-Chips, eines Antigens (als Positivkontrolle) zu erkennen, um eine Orientierung des Antigen-Chips zu bestimmen.

Gemäß einer Ausführungsform der vorliegenden Erfindung ist ein Verfahren zur Herstellung eines Antigen-Chips zur indirekten Immunofluoreszenzdiagnostik bereitgestellt, aufweisend Aufbringen von Antigen-Spots, die einen Luminophor, ein chromogenes Substrat oder einen ersten Farbstoff, insbesondere ersten Fluoreszenzfarbstoff, enthalten, auf eine ebene Substratoberfläche eines Substrats in einem für jedes Antigen vorbestimmten Muster, Fragmentieren des Substrats (z.B. in Stücke an beliebigen Stellen und/oder Bruchkurven, d.h. ein positionsungenaues Fragmentieren), um mehrere Antigen-Chips zu erhalten. Da in bevorzugten Ausführungsformen das Fragment größer ist als ein Array, ist mindestens ein kompletter Satz von Spots für einen Array auf einem Chip/Substrat vorhanden. Das Fragmentieren des Substrats in mehrere Stücke, um mehrere Antigen-Chips zu erhalten, kann mit einer gewissen Ungenauigkeit hinsichtlich der Positionierung von Bruchlinien bzw. Schnittlinien erfolgen. Eine absolute Positionierung der Antigen-Spots relativ zu Rändern des Antigen-Chips braucht nicht erforderlich sein, um ein Verfahren zum Detektieren von Antikörpern durchzuführen, denn während des Detektionsverfahrens werden die Positionen der Antigen-Spots bestimmt, ohne eine absolute vorbestimmte Positionierung der Antigen-Spots vorauszusetzen. In bevorzugten Ausführungsformen mit mindestens zwei Referenz-Antigenspots bzw. Referenz-Antigenspotreihen ist es möglich, das Antigen-Substrat (z.B. während des Herstellungsprozesses) um 180° zu drehen und aufgrund der mindestens zwei Referenz-Antigenspots bzw. Referenz-Antigenspotreihen mit z.B. unterscheidbaren Spotabständen/Muster eine eindeutige Zuordnung der Antigenspots vorzunehmen. Damit können relativ ungenaue Herstellungsverfahren oder Fragmentierungsverfahren zur Anwendung kommen, was Aufwand, Kosten und Komplexität der Herstellung vereinfachen kann.

Das Aufbringen der Antigen-Spots kann mittels eines piezoelektrischen Mikrodosiergeräts durchgeführt werden, und die Substratoberfläche kann insbesondere eine Oberfläche eines Glas-Substrates oder eines mit einer Membran und/oder Folie beschichteten Glas-Substrates sein. Damit ist die Herstellung mit herkömmlichen Grundmaterialien und Herstellungsmaschinen durchführbar.

Gemäß einer Ausführungsform der vorliegenden Erfindung ist ein Verfahren zum Detektieren von Antikörpern in einer Probe, insbesondere durch indirekte Immunofluoreszenz, bereitgestellt, wobei das Verfahren aufweist: Bereitstellen von Antigen-Spots, die einen Luminophor, ein chromogenes Substrat oder ersten Farbstoff, insbesondere ersten Fluoreszenzfarbstoff, enthalten und auf einem ebenen Substrat in einem vorbestimmten Muster angeordnet sind, Inkubieren der Antigen-Spots mit der Probe; Inkubieren der Antigen-Spots mit einem sekundären Antikörper, der mit einem zweiten Luminophor, einem zweiten chromogenen Substrat, Enzym oder zweiten Fluoreszenzfarbstoff markiert ist; Beleuchten der Antigen-Spots mit Beleuchtungslicht, insbesondere Anregungslicht, um eine erste Strahlung (z.B. von den Spots, insbesondere dem ersten Farbstoff, rückgestreutes Beleuchtungslicht) auszulösen, insbesondere um erste Fluoreszenz des ersten Fluoreszenzfarbstoffes (z.B. Rotkanal), anzuregen und zweite Fluoreszenz des zweiten Fluoreszenzfarbstoffes (z.B. Grünkanal) anzuregen, Detektieren der ersten Strahlung, insbesondere der ersten Fluoreszenz, durch Aufnehmen eines ersten Bildes mit einer ersten Kamera und Aufnehmen eines zweiten Bildes mit der ersten oder einer zweiten Kamera, um Positionen der Antigen-Spots zu bestimmen; Zuordnen der Positionen der Antigen-Spots zu jeweiligen Antigenen anhand des vorbestimmen Musters; und Detektieren der zweiten Chemilumineszenz oder Fluoreszenz im zweiten Bilde, um an Antigen-Spots gebundene Antikörper (insbesondere quantitativ) zu erkennen.

In einer bevorzugten Ausführungsform des Verfahrens sind die erste und zweite Fluoreszenz über die gleiche Wellenlänge anregbar sind. In weiter bevorzugten Ausführungsformen wird die erste Fluoreszenz durch Anregung von DY-521-XL und die zweite Fluoreszenz durch Anregung von Fluoresceinisothiocyanat (FITC) erzeugt.

Das Verfahren kann mittels einer Vorrichtung zum Detektieren von Antikörpern gemäß einer Ausführungsform durchgeführt werden. Ferner kann der zweite Fluoreszenzfarbstoff derart ausgewählt sein, dass er mit Anregungslicht, welches zur Anregung des ersten Fluoreszenzfarbstoffes geeignet ist, angeregt werden kann, jedoch in einem zweiten Wellenlängenbereich fluoresziert, welcher im Wesentlichen verschieden ist von einem ersten Wellenlängenbereich, in welchem der erste Fluoreszenzfarbstoff fluoresziert. Die Probe kann z.B. ein Serum, ein Plasma oder ein anderes Probenmaterial sein, beispielsweise menschlichen Ursprungs. Das erste Bild kann durch mehrere erste Teilbilder (zum Beispiel vier Teilbilder) zusammengesetzt werden, die verschiedene Teilbereiche des Antigen-Chips abbilden. Ebenso kann das zweite Bild durch mehrere Teilbilder (zum Beispiel vier Teilbilder) zusammengesetzt werden, die nacheinander von verschiedenen Teilbereichen des Antigen-Chips aufgenommen worden sind. In anderen Ausführungsformen wird jeweils nur ein einziges erstes Bild von dem gesamten Antigen-Chip aufgenommen und es wird nur ein einziges zweites Bild von dem gesamten Antigen-Chip aufgenommen.

In bevorzugten Ausführungsformen kann das Verfahren zum Detektieren von Antikörpern einen sogenannten "Cut-off-Kalibrator" und dessen Messung umfassen. Der Cut-off-Kalibrator wird als Referenz für die Unterscheidung von "positiven" von "negativen" Signalen verwendet. Die Art des Cut-off-Kalibrators kann den gegebenen Parametern des Nachweissystems angepasst sein und z.B. entsprechend der Art des Antigens, Antikörpers und des Farbstoffes, insbesondere des Fluoreszenzfarbstoffes, verändert werden. In weiter bevorzugten Ausführungsformen kann der Cut-off-Kalibrator ein externer Kalibrator sein. Hierbei kann es sich um eine Kalibrationsprobe handeln, deren Konzentration z.B. in etwa in der Höhe des Cut-offs der zu messenden Probe ist. Diese Kalibrationsprobe kann auf einem separaten Array (derselben Arraycharge) parallel inkubiert werden und auf die mit den Proben erzielten Intensitäten relativ bezogen werden. In alternativen Ausführungsformen kann der Cut-off-Kalibrator ein interner Kalibrator sein, wobei der Kalibrator hier ein entsprechendes Kalibratormaterial umfassender Spot auf dem erfindungsgemäßen Antigen-Chip ist, wie z.B. ein human-IgG-Spot.

Gemäß einer Ausführungsform der vorliegenden Erfindung ist ein Kit zur Verwendung in einem Verfahren zum Detektieren von Antikörpern in einer Probe, insbesondere durch indirekte Immunofluoreszenz, bereitgestellt. Das Kit weist einen Antigen-Chip gemäß einem der vorangehend beschriebenen Ausführungsformen und eine Sonde, insbesondere sekundären Antikörper, auf, die mit einem zweiten Luminophor, zweiten chromogenen Substrat, Enzym oder zweiten Fluoreszenzfarbstoffes markiert ist. Der erste und der zweite Fluoreszenzfarbstoff des Kits können (müssen jedoch nicht) durch im Wesentlichen denselben Wellenlängenbereich angeregt werden. In bevorzugten Ausführungsformen sind der erste und der zweite Farbstoff jeweils ein Fluoreszenzfarbstoff und die erste und die zweite Fluoreszenz sind über die gleiche Wellenlänge anregbar, wobei sich die Emissionsspektren, insbesondere die Emissionsmaxima, unterscheiden. In bevorzugten Ausführungsformen sind die Emissionsmaxima der beiden vergleichbar anzuregenden Fluoreszenzfarbstoff mindestens 5 nm, mindestens 10 nm, mindestens 20 nm, mindestens 25 nm, mindestens 30 nm, mindestens 35 nm, mindestens 40 nm, mindestens 45 nm, mindestens 50 nm, mindestens 55 nm oder minstens 60 nm voneinander entfernt. In weiter bevorzugten Ausführungsformen wird die erste Fluoreszenz durch Anregung von DY-521-XL und die zweite Fluoreszenz durch Anregung von Fluoresceinisothiocyanat (FITC) erzeugt.

Ausführungsformen der vorliegenden Erfindung ermöglichen, durch einen ersten (Fluoreszenz-) Farbstoff, chromogenes Substrat oder Luminophor einen Spot eines Arrays zu lokalisieren und diese Lokalisierung für die Messung eines Signals, z.B. eines zweiten Fluorophors, zu nutzen.

Ein Objektträger kann anstatt einem Antigen-Chip auch eine Vielzahl von Antigen-Chips umfassen (insbesondere 2, 3, 4, 5, 6, 7, usw. Antigen-Chips) und die hier für einen einzelnen Antigen-Chip beschrieben Antigen-Spot Eigenschaften sowie das vorbestimmte Muster der Antigen-Spots kann auf die Vielzahl der Antigen-Chips (für jeden einzeln oder in ihrer Gesamtheit) übertragen werden. Beispielsweise kann auf einem Objektträger, der zwei erfindungsgemäße Antigen-Chips umfasst, der erste Antigen-Chip eine Inkubationskontrolle und der zweite Antigen-Chip eine Kalibrierspotlinie umfassen. Die hierin für einen Antigen-Chip beschriebenen Spot-Anordnungen und -Eigenschaften kann also durch die Vielzahl der Antigen-Chips in ihrer Gesamtheit ausgeführt sein, ohne dass jeder einzelne Antigen-Chip der Vielzahl von Antigen-Chips diese Spot-Anordnungen und -Eigenschaften für sich alleine ausführt.

Ausführungsform der vorliegenden Erfindung ermöglichen ferner (separat oder in Kombination) eine oder mehrere Antigen-Spot-Reihen von weiteren Spot-Reihen eines Antigen-Chips abzugrenzen (z.B. über den Abstand der einzelnen Spots), um eindeutig die Orientierung des Chips und somit auch die Art der Antigen-Spot-Reihen bestimmen zu können (z.B. wenn der Chip Spot-Reihen mit verschiedenen Antigenen enthält).

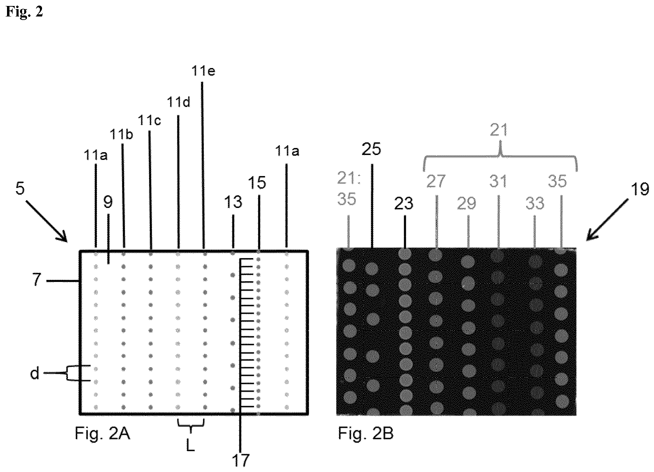

Ein Antigen-Chip kann mindestens eine Antigen-Spot-Reihe bzw. mindestens eine Kalibrier-Spot-Reihe, bevorzugt mindestens zwei Antigen-Spot-Reihen oder Kalibrier-Spot-Reihen, umfassen, die von den anderen Spot-Reihen durch mindestens eine der folgenden Markierungsmöglichkeiten unterschieden werden können:
- Art des Farbstoffs (z.B. hinsichtlich der reflektierten oder angeregten Emission);
- Intensität (Menge bzw. Konzentration) des Farbstoffs;
- Abstand der Antigen-Spot-Reihe zu anderen Antigen-Spot-Reihen (Abstand L in Fig. 2A);
- Abstand der Antigen-Spots innerhalb einer Reihe (Abstand d in Fig. 2A);
- Form (z.B. kreisförmig, rechteckig, quadratisch, oval, Ausgestaltung als durchgehende Linie) der Antigen-Spots; und/oder
- Umfang (bzw. Ausdehnung/Durchmesser) der Antigen-Spots.

Dadurch kann der technische Vorteil erreicht werden, dass die Herstellung des Antigen-Chips ein weniger genaues Positionieren der Antigen-Spots bzw. weniger genaues Fragmentieren (z.B. Brechen des Glases) tolerieren kann, da bei Verwendung des Antigen-Chip in einem Scanner- bzw. Kamerasystem, die Identifikation und Lokalisation der Antigen-Spots durch Auswertung des ersten Bildes erfolgt, ohne dass eine vorbestimmte Positionierung des Antigen-Spots und/oder des Substrates des Antigen-Chips relativ zum Kamerasystem erforderlich ist.

Ausführungsformen der vorliegenden Erfindung werden nun mit Bezug auf die beiliegenden Figuren erläutert. Die Erfindung ist nicht auf die illustrierten oder beschriebenen Ausführungsformen beschränkt.
Fig. 1 illustriert in einer Draufsicht einen Objektträger mit mehreren Antigen-Chips gemäß einer Ausführungsform der vorliegenden Erfindung;
Fig. 2A illustriert in einer schematischen Darstellung einen Antigen-Chip mit Antigen-Spots in einem vorbestimmten Muster gemäß einer Ausführungsform der vorliegenden Erfindung;
Fig. 2B illustriert ein erstes Bild zur Lokalisierung von Antigen-Spots, welches gemäß einer Ausführungsform ausgewertet und aufgenommen wurde;
Fig. 3 illustriert schematisch eine Vorrichtung zum Detektieren von Antikörpern in einer Probe gemäß einer Ausführungsform der vorliegenden Erfindung;
Fig. 4 und 5 illustrieren ein erstes Bild bzw. ein zweites Bild, wie sie gemäß Ausführungsformen der vorliegenden Erfindung aufgenommen und ausgewertet werden;
Fig. 6 illustriert ein Emissionsspektrum einer Beleuchtungsquelle einer Vorrichtung gemäß einer Ausführungsform der vorliegenden Erfindung;
Fig. 7 illustriert ein Anregungsspektrum und ein Emissionsspektrum eines Farbstoffes, welcher in Ausführungsformen der vorliegenden Erfindung eingesetzt wird; und
Fig. 8 illustriert Emissionsspektren zweier Fluoreszenzfarbstoffe, wie sie in Ausführungsformen der vorliegenden Erfindung eingesetzt werden können.

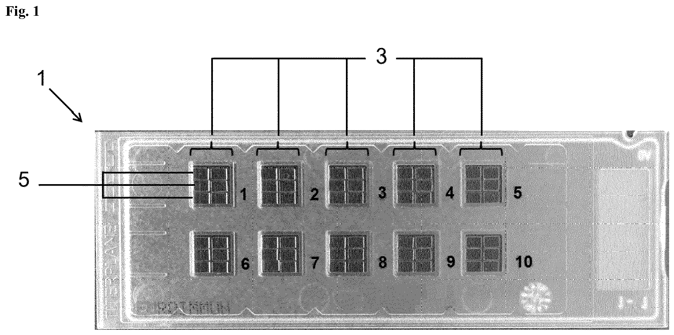

Der in Fig. 1 in Draufsicht illustrierte Objektträger 1 umfasst zehn Objektträgerfelder 3, welche in zwei Reihen zu je fünf Feldern 3 angeordnet sind und mit 1 bis 10 durchnummeriert sind. Jedes Feld 3 umfasst in Fig. 1 sechs Antigen-Chips 5 (in anderen bevorzugten Ausführungsformen können es 1, 2, 3, 4, oder 5 Antigen-Chips 5 sein), welche gemäß einer Ausführungsform der vorliegenden Erfindung bereitgestellt sind. Ein Antigen-Chip 5 ist zum Beispiel schematisch in Fig. 2A dargestellt. Der Antigen-Chip 5 weist ein Substrat 7 auf, welches eine ebene Substratoberfläche 9 hat. Auf der Substratoberfläche 9 sind Antigen-Spots 11a, 11b, 11c, 11d, 11e in einem bestimmten Muster aufgebracht, wobei die Antigen-Spots 11a, 11b, 11c, 11d, 11e jeweils einen ersten Fluoreszenzfarbstoff enthalten, wobei die Linie 11a in doppelter Ausführungsform auf dem Antigen-Chip aufgebracht ist. In anderen Ausführungsformen ist auch eine einfache Ausführungsform der Linie 11a möglich. Auf der Substratoberfläche 9 ist ferner die Linie 13 mit einem im Vergleich zu den Linien 11a, 11b, 11c, 11d, 11e weiteren Dotabstand aufgetragen, welche erlaubt zu bestimmen, ob der Antigen-Chip 5 mit einer Probe inkubiert wurde. Ferner ist auf dem Antigen-Chip 5 eine Linie 15 aufgebracht, welche durch eine Mehrzahl von Kalibrierungsspots 17 gebildet ist, die einen primären Antikörper in gleichen oder verschiedenen Mengen bzw. Konzentrationen enthalten.

Die Antigen-Spots 11a sind entlang einer Linie angeordnet und enthalten jeweils das gleiche Antigen. Ebenso sind die Antigen-Spots 11b, 11c, 11d und 11e jeweils in einer Linie angeordnet und enthalten für jede Linie ein gleiches Antigen. In einem Antigen-Chip 5 können die in Linien angeordneten Spots für ein Antigen mehr oder weniger Spots aufweisen als abgebildet, zum Beispiel zwischen zwei und zwanzig Spots für jedes Antigen, insbesondere zwischen vier und zehn Spots für jedes Antigen. Antigene in einer Linie von Antigen-Spots können in einer gleichen oder in einer verschiedenen Konzentration oder Mengen in den jeweiligen Antigen-Spots enthalten sein. Eine Linie von Antigen-Spots kann durch einen Spotabstand d charakterisiert sein, sowie durch eine Spotgröße und/oder eine Spotform. Die Spotabstände d können für Linien, welche jeweils verschiedene Antigene enthalten, verschieden oder im Wesentlichen gleich sein. Die in Linien angeordneten Antigen-Spots 11a, 11b, 11c, 11d, 11e sind voneinander um einen bestimmten Abstand L beabstandet. Der Abstand zwischen verschiedenen Linien verschiedener Antigene kann variieren. Die Formen der Antigen-Spots der Spots 11a, 11b, 11c, 11d, 11e sind im Wesentlichen kreisförmig. Das Muster, in dem die verschiedenen Antigen-Spots 11a, 11b, 11c, 11d, 11e auf der Substratoberfläche 9 aufgebracht, insbesondere gespottet sind, ist durch den Spotabstand d (welcher in jeder der Linien verschieden sein kann oder gleich sein kann) und den Linienabstand L (welcher ebenfalls zwischen verschiedenen Linienpaaren verschieden oder gleich sein kann) sowie auch durch die Form und/oder Größe aller Antigen-Spots gegeben bzw. definiert.

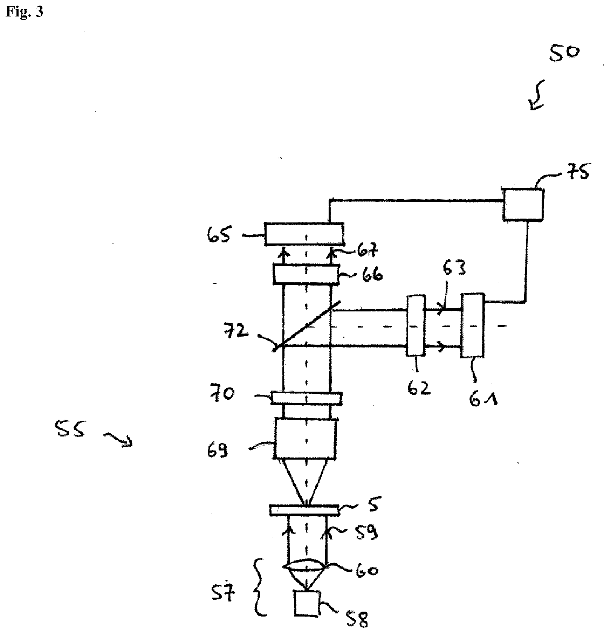

Ein Auswertesystem (wie es beispielsweise in Fig. 3 dargestellt ist) kann das Muster, in welchem die Antigen-Spots auf der Substratoberfläche aufgebracht sind, in seiner Geometrie kennen und kann auch die Belegung der einzelnen Spots mit Antigenen kennen. Eine Erkennung dieses Musters in aufgenommenen Bildern kann dann eine Zuordnung der Antigen-Spots zu bestimmten Antigenen erlauben und somit eine Zuordnung von gebundenen Antikörpern an bestimmte Antigene.

Fig. 2B illustriert schematisch ein von einer Vorrichtung (aus z.B. 4 Teilbildern) aufgenommenes Bild 19 des zum Beispiel in Fig. 2A illustrierten Antigen-Chips 5. Zur Aufnahme des Bildes 19 (z.B. eines ersten Bildes) wurde die erste Fluoreszenz erfasst, welche durch Aussendung von Licht von dem ersten Fluoreszenzfarbstoff ausgeht. Wie oben erläutert, ist der erste Fluoreszenzfarbstoff in allen Antigen-Spots 11a, 11b, 11c, 11d und 11e enthalten sowie auch in den Positivkontrolllinien 13 und in den Kalibrierungsspots 17. Die in dem Bild 19 aufgenommenen Intensitäten der ersten Fluoreszenz sind als Grautöne dargestellt, wobei hellere Grautöne eine höhere detektierte Intensität anzeigen. Die hellen Flecken bzw. Bereiche 21, 23 bzw. 25 werden im Folgenden als erste Fluoreszenzflecken bezeichnet. Wie aus Fig. 2A und 2B ersichtlich ist, erscheinen die ersten Fluoreszenzflecken 21, 23 und 25 genau an den Positionen, an denen die Antigen/Antikörper-Spots 11a, 11b, 11c, 11d, 11e bzw. die Linie 13 und 15 auf der Substratoberfläche 9 aufgebracht wurden. Ein Auswertesystem erkennt durch Vergleich mit dem vorbekannten Muster, dass beispielsweise die Spalte 35 von ersten Fluoreszenzflecken 21 von erster Fluoreszenz herrühren, welche von den Antigen-Spots 11e ausgeht. Ferner bestimmt das Auswertesystem, dass die Spalten 27, 29, 31, 33, 35 von Fluoreszenz herrühren, die von den Antigen-Spots 11a, 11b, 11c, 11d bzw. 11e ausgeht. Damit ist das Auswertesystem in der Lage, für jeden Bereich des Bildes 19 eine Zuordnung zu einem bestimmten Antigen bzw. der Positivkontrolle oder der Kalibrierung vorzunehmen.

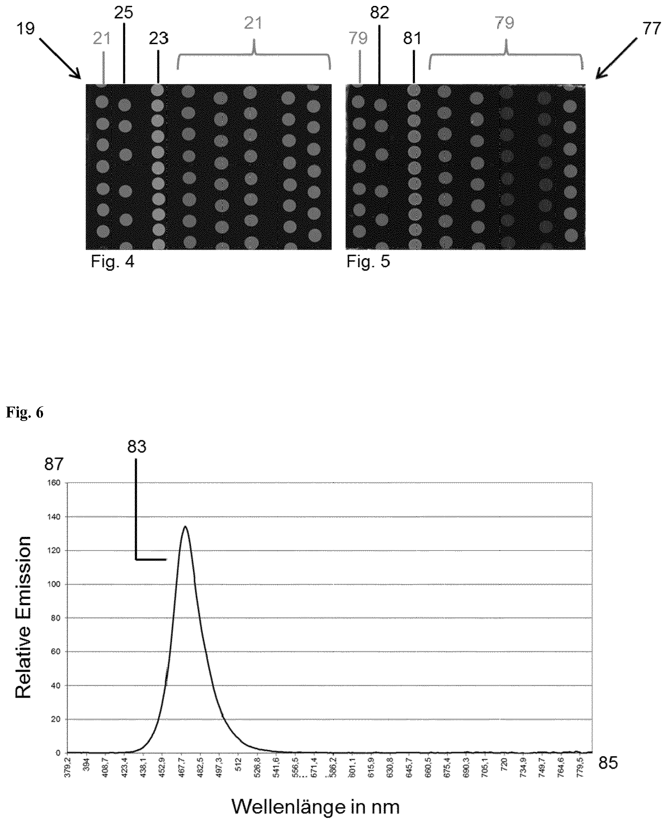

Fig. 3 illustriert in schematischer Darstellung eine Vorrichtung 50 zum Detektieren von Antikörpern in einer Probe gemäß einer Ausführungsform der vorliegenden Erfindung. Die Vorrichtung 50 umfasst dabei einen Antigen-Chip 5, beispielsweise den in Fig. 2A illustrierten Antigen-Chip 5. Ferner umfasst die Vorrichtung ein Fluoreszenzmikroskop 55, welches eine Beleuchtungsquelle 57 aufweist, die ausgebildet ist, Anregungslicht 59 zu erzeugen und die angeordnet ist, den Antigen-Chip 5 mit dem Anregungslicht 59 zu bestrahlen. Im Detail umfasst die Beleuchtungsquelle 57 eine LED 58 und eine Abbildungsoptik 60, um den Antigen-Chip 5 vollständig oder zumindest teilweise zu beleuchten. Ferner umfasst das Fluoreszenzmikroskop 55 eine erste Kamera 61, die zum Aufnehmen eines ersten Bildes (zum Beispiel des Bildes 19 in Fig. 2B) durch Detektion von von der Probe ausgehender durch das Anregungslicht 59 angeregter erster Fluoreszenz 63 ausgebildet ist. Ferner umfasst das Fluoreszenzmikroskop 55 eine zweite Kamera 65, die zum Aufnehmen eines zweiten Bildes (siehe zum Beispiel Fig. 5) durch Detektion von von der Probe ausgehender durch das Anregungslicht 59 angeregter zweiter Fluoreszenz 67 ausgebildet ist. Das Fluoreszenzmikroskop umfasst ferner ein Objektiv 69 (mit einer oder mit mehreren Linsen), welches zum Erzeugen eines vergrößerten Bildes des Antigen-Chips 5 ausgebildet ist.

Nachdem der Antigen-Chip 5 mit einer Probe inkubiert worden ist, die potenziell Antikörper enthält, welche an einen oder mehrere der Antigen-Spots binden, wird die Probe mit einem zweiten fluoreszenzfarbstoffmarkierten Antikörper inkubiert. Durch das Anregungslicht 59 wird sowohl der erste Fluoreszenzfarbstoff (welcher in den Antigen-Spots enthalten ist) als auch der zweite Fluoreszenzfarbstoff (der zum Beispiel an einen sekundären Antikörper gebunden ist) zur Anregung von erster bzw. zweiter Fluoreszenz angeregt, deren Licht gleichzeitig durch das Objektiv 69 tritt. Stromabwärts des Objektivs ist ein Anregungslichtfilter 70 angeordnet, um wesentliche Teile des Anregungslichts 59 herauszufiltern, jedoch Licht der ersten Fluoreszenz 63 und auch Licht der zweiten Fluoreszenz 67 im Wesentlichen passieren zu lassen. Das Licht der ersten Fluoreszenz und zweiten Fluoreszenz trifft sodann auf einen halbdurchlässigen Spiegel 72, der einen Teil reflektiert, um auf ein erstes Filter 62 zu treffen und dieses zu durchsetzen. Das erste Filter 62 ist in einem ersten Strahlengang stromaufwärts der ersten Kamera 61 angeordnet und ausgebildet, das Anregungslicht 59 und Licht der zweiten Fluoreszenz 67 im Wesentlichen zu eliminieren. Somit trifft auf die erste Kamera 61 im Wesentlichen nur das Licht 63 der ersten Fluoreszenz. Ein Teil des Fluoreszenzlichts wird von dem halbdurchlässigen Spiegel 72 durchgelassen und durchsetzt ein zweites Filter 66, welches in einem zweiten Strahlengang stromaufwärts der zweiten Kamera 65 angeordnet und ausgebildet ist, das Anregungslicht 59 sowie das Licht der ersten Fluoreszenz 63 im Wesentlichen zu eliminieren. Somit trifft auf die zweite Kamera 65 im Wesentlichen nur das Licht 67 der zweiten Fluoreszenz.

Die Vorrichtung 50 umfasst ferner ein Auswertesystem 75, welches von der ersten Kamera 61 und der zweiten Kamera 65 aufgenommene erste Bilder bzw. zweite Bilder empfängt und ausgebildet ist, diese auszuwerten. Dazu werden basierend auf dem ersten Bild (zum Beispiel Bild 19 in Fig. 2B) Positionen der Antigen-Spots bestimmt und die Positionen der Antigen-Spots werden jeweils Antigenen anhand des vorbestimmten Musters zugeordnet. Ausgehend von den in dem ersten Bild lokalisierten ersten Fluoreszenzflecken 21, 23 und 25 können dann in dem zweiten Bild (zum Beispiel Fig. 4 oder 5) erkannte Intensitäten bestimmten Antigenen zugeordnet werden, um somit gebundene Antikörper qualitativ zu detektieren oder zumindest semi-quantitativ zu erkennen.

Fig. 4 und 5 illustrieren Beispiele eines ersten Bildes 19 bzw. eines zweiten Bildes 77, welche von dem Auswertesystem 75 erhalten und verarbeitet werden. Die von dem Auswertesystem 75 in dem ersten Bild 19 erkannten ersten Fluoreszenzflecken 21, 23 und 25 werden in ihrer Position und Größe bestimmt. Die in dem ersten Bild 19 bestimmten Positionen und Größen der ersten Fluoreszenzflecken werden dann auf das zweite Bild 77 übertragen, um die Bereiche zu definieren, an denen ursprünglich auf dem Antigen-Chip Antigen-Spots mit bekannten Antigenen vorhanden waren. Diese Bereiche enthalten dann zweite Fluoreszenzflecken 79, 81, 82 deren Intensitäten (das heißt Intensität von Licht der zweiten Fluoreszenz) von dem Auswertesystem 75 bestimmten Antigenen zugeordnet werden. Um eine Quantifizierung vornehmen zu können, kann das Auswertesystem ferner ausgebildet sein, in den zweiten Bildintensitäten von zumindest zwei, insbesondere mindestens drei, Kalibrierungsspots ausgehende zweite Fluoreszenz zu bestimmen, um eine Kalibrierung durchzuführen. Die Kalibrierungsspots 17 des Antigen-Chips 5 in Fig. 2A können zum Beispiel einen primären Antikörper (zum Beispiel IgG) in gleichen oder verschiedenen Mengen bzw. Konzentrationen enthalten. Auch an diesen primären Antikörper werden nach Inkubation mit dem mit dem zweiten Fluoreszenzfarbstoff markierten sekundären Antikörper die sekundären Antikörper binden, so dass auch von diesen Bereichen zweite Fluoreszenz ausgehen wird, welche abhängig ist von der ursprünglichen Konzentration des primären Antikörpers.

Insbesondere kann eine automatische Spotidentifizierung im Rotkanal und eine Zuordnung der unterschiedlichen Antigene von dem Auswertesystem durchgeführt werden. Eine automatische qualitative bzw. quantitative Auswertung der Antigen-Reaktivität kann im Grünkanal erfolgen. Eine semi-quantitative Auswertung kann mittels standardisierter Kontrollseren und einer Angabe der Fluoreszenzintensitätswerte für jedes Antigen durchgeführt werden. Es kann auch eine Kalibrierungskurve für die Grünfluoreszenz anhand von humanem IgG-Dots erstellt werden.

Ausführungsformen der vorliegenden Erfindung stellen auch ein Herstellungsverfahren eines Antigen-Chips bereit, wobei Antigen-Spots, die einen Fluoreszenzfarbstoff enthalten, auf eine ebene Substratoberfläche eines Substrats in einem für jedes Antigen vorbestimmten Muster aufgebracht werden und das Substrat sodann fragmentiert wird (ohne eine genaue Positionierung der Trennungslinien zu erfordern), um mehrere Antigen-Chips zu erhalten. Die Antigene können mit einem piezoelektrischen Mikrodosiergerät zum Beispiel auf Glas oder auf glasbeschichteter Membran/Folie erfolgen. Eine Patientenprobe (zum Beispiel Serum oder Plasma) kann inkubiert werden.

Der Antigen-Chip kann zum Beispiel eine Größe von 1,4 mm x 2 mm haben. Herkömmlicherweise ist eine positionsgenaue Aufbringung von Antigen-Spots auf einer Substratoberfläche problematisch. Diese Notwendigkeit kann durch Ausführungsformen der vorliegenden Erfindung eliminiert werden, da die Positionen der Antigen-Spots innerhalb des Verfahrens selbst bestimmt werden.

Fig. 6 illustriert beispielhaft ein Emissionsspektrum 83 einer Beleuchtungsquelle 58 gemäß einer Ausführungsform der vorliegenden Erfindung, wobei auf einer Abszisse 85 die Wellenlänge und auf einer Ordinate 87 die relative Emission aufgetragen sind. Das Spektrum 83 zeigt eine Emission zwischen 440 nm und 520 nm mit einem Maximum bei etwa 470 nm. Ein solches Emissionsspektrum kann zur Erzeugung von Anregungslicht 59 zur Anregung von erster Fluoreszenz und zweiter Fluoreszenz verwendet werden.

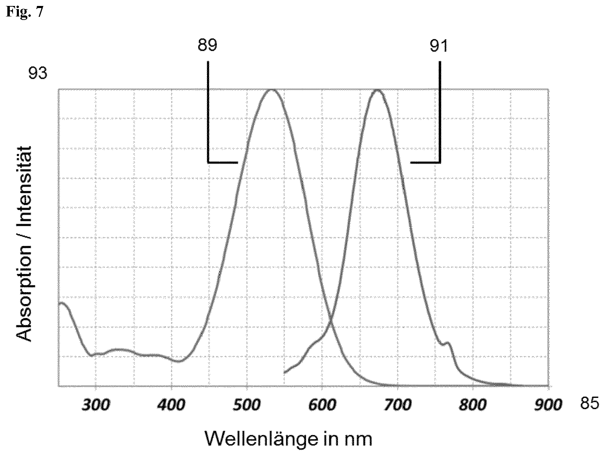

Fig. 7 illustriert ein Anregungsspektrum 89 bzw. ein Emissions- oder Fluoreszenzspektrum 91 eines ersten Fluoreszenzfarbstoffes, wie er in Ausführungsformen der vorliegenden Erfindung eingesetzt werden kann, wobei auf der Abszisse 85 die Wellenlänge und auf der Ordinate 93 die Absorbanz bzw. die Emissionsintensität aufgetragen sind. Eine Anregung erfolgt ab ca. 450 nm bis etwa 605 nm mit einem Maximum bei etwa 520 nm. Die Emission erfolgt ab etwa 555 nm bis etwa 800 nm mit einem Maximum bei etwa 670 nm. Bei dem hier in Fig. 7 illustrierten Beispiel handelt es sich um die Absorption bzw. Emission des Farbstoffes DY521XL (z.B. erster Fluoreszenzfarbstoff).

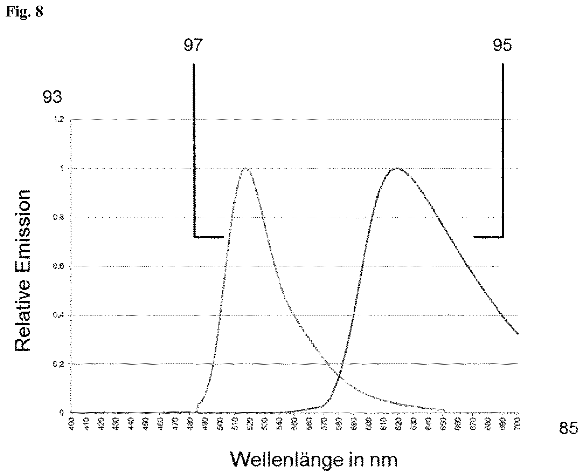

Fig. 8 illustriert beispielhaft Emissions- oder Fluoreszenzspektren 95 und 97 eines ersten Fluoreszenzfarbstoffes bzw. eines zweiten Fluoreszenzfarbstoffes, wobei wiederum auf der Abszisse 85 die Wellenlänge und auf der Ordinate 93 die Emissionsintensität aufgetragen sind. Wie aus Fig. 8 ersichtlich ist, überlappen die Emissionen der ersten Fluoreszenz 95 und der zweiten Fluoreszenz 97 nur in einem geringen Bereich, sind also spezifisch für einerseits Detektion der Spotorte und andererseits Detektion der gebundenen sekundären Antikörper. Die Fluoreszenzemission 97 kann zum Beispiel durch FITC realisiert werden, die Fluoreszenz 95 kann zum Beispiel durch PI (Propidiumiodid) realisiert werden.

Ausführungsformen der vorliegenden Erfindung können ohne einen Scanprozess zur Aufnahme der ersten Bilder und zweiten Bilder auskommen. Die Vergrößerung, welche durch das Objektiv 69 bereitgestellt ist, kann zum Beispiel zwischen 5, 8, 10, 15, 20 oder 40 Mal liegen. Gemäß Ausführungsformen der vorliegenden Erfindung ist pro Antigen-Spot etwa 300 pl einer Antigenlösung einer Konzentration von etwa 0,2 mg/ml ausreichend. Eine Patientenprobe von etwa 0,3 bis 3µl pro Feld (das heißt sechs Antigen-Chips) kann verdünnt inkubiert werden.

## Patentansprüche

1. Antigen-Chip (5), aufweisend:
eine ebene Substratoberfläche (9);
Antigen-Spots (11a, ..., 11e), die auf der Substratoberfläche in einem vorbestimmten Muster aufgebracht sind und einen ersten Farbstoff, ein chromogenes Substrat oder ein Luminophor, insbesondere ersten Fluoreszenzfarbstoff, enthalten.

2. Antigen-Chip gemäß dem vorangehenden Anspruch, wobei Antigen-Spots (11a, ..., 11e), die verschiedene Antigene enthalten, verschiedene Mengen bzw. Konzentrationen des ersten Luminophors, chromogenen Substrats oder Farbstoffes, insbesondere Fluoreszenzfarbstoffes, derart enthalten,
dass davon zurückgeworfene erste Strahlung, insbesondere angeregte erste Fluoreszenz, im Wesentlichen gleiche Intensität hat oder
dass von Antigen-Spots verschiedener Linien zurückgeworfene erste Strahlung, insbesondere angeregte erste Fluoreszenz, im Wesentlichen unterschiedliche Intensität hat, um die Linien unterscheiden zu können.

3. Antigen-Chip gemäß einem der vorangehenden Ansprüche, wobei
(a) Antigen-Spots (11a, ..., 11e) eines gleichen Antigens jeweils in einer Linie, insbesondere mit einem vorbestimmten Spotabstand (d), angeordnet sind, wobei verschiedenen Antigenen zugeordnete Linien insbesondere einen vorbestimmen Linienabstand (L) und/oder Anordnung aufweisen,
wobei ferner insbesondere der Linienabstand zweier Linien größer ist als der Spotabstand innerhalb einer Linie, und/oder
(b) Antigen-Spots (11a, ..., 11e) eines gleichen Antigens jeweils in einer Linie angeordnet sind, wobei mindestens zwei Linien einen unterscheidbaren vorbestimmten Spotabstand (d) aufweisen.

4. Antigen-Chip gemäß einem der vorangehenden Ansprüche, wobei auf der Substratoberfläche (9) ferner zumindest zwei, insbesondere mindestens drei, Kalibrierungsspots (17) aufgebracht sind, die einen primären Antikörper in jeweils verschiedenen Mengen bzw. Konzentrationen enthalten.

5. Antigen-Chip gemäß einem der vorangehenden Ansprüche, wobei auf dem Substrat ferner zumindest eine Linie (13) eines Antigens, insbesondere eine Linie mit einem größeren oder geringeren Dotabstand im Vergleich zu den anderen Linien des Antigen-Chips, als Positivkontrolle aufgebracht ist.

6. Antigen-Chip gemäß einem der vorangehenden Ansprüche, wobei
(a) die Substratoberfläche (9) zur Fokussierung mikrostrukturiert ist und insbesondere die Substratoberfläche (9) eine Größe zwischen 1 mm x 1 mm und 2 mm x 4 mm aufweist, und/oder
(b) der Antigen-Chip einen Cut-off-Kalibrator aufweist.

7. Vorrichtung (50) zum Detektieren von Antikörpern in einer Probe, aufweisend:
einen Antigen-Chip (5) gemäß einem der vorangehenden Ansprüche; und
ein Scanner- und/oder Kamerasystem, insbesondere ein Fluoreszenzmikroskop (55) mit:
einer Beleuchtungsquelle (57), die ausgebildet ist, Beleuchtungslicht, insbesondere Anregungslicht (59), zu erzeugen, und die angeordnet ist, den Antigen-Chip (5) zu beleuchten, insbesondere zu bestrahlen;
einer ersten Kamera (61), die zum Aufnehmen eines ersten Bildes (19) durch Detektion von von den Antigen-Spots ausgehender durch das Beleuchtungslicht hervorgerufener erster Strahlung, insbesondere durch das Anregungslicht angeregter erster Fluoreszenz, ausgebildet ist und/oder zum Aufnehmen eines zweiten Bildes (77) durch Detektion von von der Probe angeregter Chemilumineszenz oder durch das Beleuchtungslicht, insbesondere Anregungslicht, angeregter zweiter Fluoreszenz ausgebildet ist,
wobei das Scanner- und/oder Kamerasystem, insbesondere das Fluoreszenzmikroskop (55), ferner bevorzugt aufweist:
eine zweite Kamera (65), die zum Aufnehmen eines zweiten Bildes (77) durch Detektion von von der Probe angeregter Chemilumineszenz oder durch das Beleuchtungslicht, insbesondere Anregungslicht, angeregter zweiter Fluoreszenz ausgebildet ist, insbesondere wenn die erste Kamera nicht ausgebildet ist die zweite Fluoreszenz zu detektieren.

8. Vorrichtung gemäß dem vorangehenden Anspruch, ferner aufweisend:
ein erstes Filter (62), welches in einem ersten Strahlengang stromaufwärts der ersten Kamera (61) angeordnet ist und ausgebildet ist, Anregungslicht und/oder Licht der zweiten Fluoreszenz im Wesentlichen zu eliminieren;
insbesondere ferner aufweisend:
ein zweites Filter (66), welches in einem zweiten Strahlengang stromaufwärts der zweiten Kamera (65) angeordnet ist und ausgebildet ist, Anregungslicht und/oder Licht der ersten Fluoreszenz im Wesentlichen zu eliminieren.

9. Vorrichtung gemäß einem der Ansprüche 7 oder 8, ferner ausweisend:
ein Auswertesystem (75), welches ausgebildet ist:
basierend aus dem ersten Bild Positionen der Antigen-Spots zu bestimmen;
die Positionen der Antigen-Spots jeweiligen Antigenen anhand des vorbestimmten Musters zuzuordnen; und/oder
an Antigen-Spots gebundene Antikörper basierend auf dem zweiten Bild, insbesondere zumindest semi-quantitativ, zu erkennen.

10. Vorrichtung gemäß einem der Ansprüche 7 bis 9, wobei das Auswertesystem ferner ausgebildet ist:
das zweite Bild auszuwerten, um Intensitäten der von den einzelnen Antigen-Spots ausgesandter zweiter Fluoreszenz zu bestimmen, und
insbesondere Intensitäten mehrerer Antigen-Spots, die das gleiche Antigen enthalten, zu mitteln.

11. Vorrichtung gemäß einem der Ansprüche 7 bis 10, wobei das Auswertesystem ferner ausgebildet ist:
in dem zweiten Bild Intensitäten von von zumindest einem, bevorzugt mindestens zwei, insbesondere mindestens drei, Kalibrierungsspot(s) (17) ausgehender zweiter Fluoreszenz zu bestimmen, um eine Kalibrierung durchzuführen, wobei die Kalibrierungsspots einen primären Antikörpers in verschiedenen Mengen bzw. Konzentrationen enthalten.

12. Vorrichtung gemäß einem der Ansprüche 7 bis 11, wobei das Auswertesystem ferner ausgebildet ist:
in dem ersten oder zweiten Bild zumindest eine Reihe von Antigen-Spots (11a, ..., 11e) und/oder eine Linie (13, 15), bevorzugt mindestens eine Reihe und/oder Linie mit einem größeren und geringeren Dotabstand im Vergleich zu den anderen Reihen und/oder Linien, besonders bevorzugt zwei Linien und/oder mit einem größeren und geringeren Dotabstand, wobei eine Linie und/oder Reihe als durchgehende Linie ausgeprägt ist, zu erkennen, um eine Orientierung des Antigen-Chips zu bestimmen und eine Zuordnung der Reihen und Antigene zueinander zu treffen.

13. Verfahren einer Herstellung eines Antigen-Chips (5) zur indirekten Immunofluoreszenzdiagnostik, aufweisend:
Aufbringen von Antigen-Spots (11a, ..., 11e), die einen ersten Farbstoff, chromogenes Substrat oder Luminophor, insbesondere ersten Fluoreszenzfarbstoff, enthalten, auf eine ebene Substratoberfläche (9) eines Substrats (7) in einem für jedes Antigen vorbestimmten Muster,
Fragmentieren des Substrats (7), insbesondere an beliebigen Stellen, um mehrere Antigen-Chips (5) zu erhalten.

14. Verfahren gemäß dem vorangehenden Anspruch, wobei das Aufbringen der Antigen-Spots mittels eines piezoelektrischen Mikrodosiergerät durchgeführt wird,
wobei die Substratoberfläche (9) insbesondere eine Oberfläche eines Glas-Substrates oder eines mit einer Membran und/oder Folie beschichteten Glas-Substrates ist.

15. Verfahren zum Detektieren von Antikörpern in einer Probe, insbesondere durch indirekte Immunofluoreszenz, wobei das Verfahren aufweist:
Bereitstellen von Antigen-Spots (11a, ..., 11e), die einen ersten Farbstoff, chromogenes Substrat oder Luminophor, insbesondere ersten Fluoreszenzfarbstoff, enthalten und auf einem ebenen Substrat (7) in einem vorbestimmen Muster angeordnet sind;
Inkubieren der Antigen-Spots mit der Probe;
Inkubieren der Antigen-Spots mit einem sekundären Antikörper, der mit einem zweiten Luminophor, zweiten chromogenem Substrat, Enzym oder zweiten Fluoreszenzfarbstoff markiert ist;
Beleuchten der Antigen-Spots (11a, ..., 11e) mit Beleuchtungslicht, insbesondere Anregungslicht (59), um eine erste Strahlung auszulösen, insbesondere um erste Fluoreszenz (63) des ersten Fluoreszenzfarbstoffes anzuregen, und insbesondere zweite Fluoreszenz (67) des zweiten Fluoreszenzfarbstoffes anzuregen;
Detektieren der ersten Strahlung, insbesondere der ersten Fluoreszenz, durch Aufnehmen eines ersten Bildes (19) mit einer ersten Kamera (61), um Positionen der Antigen-Spots zu bestimmen;
Zuordnen der Positionen der Antigen-Spots zu jeweiligen Antigenen anhand des vorbestimmten Musters; und
Detektieren der Signale des zweiten Luminophor, zweiten chromogenen Substrats oder der vom Enzym angeregten Chemilumineszenz oder der zweiten Fluoreszenz durch Aufnehmen eines zweiten Bildes (77) mit der ersten oder einer zweiten Kamera (65), um an Antigen-Spots gebundene Antikörper zu erkennen,
wobei insbesondere eine Ungenauigkeit einer Positionierung der Antigen-Spots auf dem Substrat (7) und/oder des Substrats relativ zu der ersten und/oder der zweiten Kamera toleriert wird.

16. Kit zur Verwendung in einem Verfahren zum Detektieren von Antikörpern in einer Probe, insbesondere durch indirekte Immunofluoreszenz, aufweisend:
einen Antigen-Chip gemäß einem der Ansprüche 1 bis 6; und
eine Sonde, insbesondere sekundären Antikörper, die mit einem zweiten Fluoreszenzfarbstoff, Luminophor, chromogenen Substrat oder Enzym markiert ist,
bevorzugt sind der erste und der zweite Farbstoff jeweils ein Fluoreszenzfarbstoff und die erste und die zweite Fluoreszenz sind über die gleiche Wellenlänge anregbar.
